# DEMANDE DE BREVET EUROPEEN

(11) **EP 1 723 946 A1**
(43) Date de publication de la demande: **22.11.2006**
(21) Numéro de dépôt: 06113925.9
(22) Date de dépôt: 15.05.2006
(51) Int. Cl.: A61K 8/43, A61Q 5/04, C07C 279/04, C07C 279/12, C07C 279/08, C07C 323/45, C07D 239/14, C07D 233/48

(54) **Composition de défrisage des cheveux comprenant au moins une multiguanidine hors hydroxyde**

(30) Priorité: 17.05.2005 FR 0551274
(71) Demandeur: L'ORÉAL, 75008 Paris (FR)
(72) Inventeur: Malle, Gérard, 77580 Villiers S/Morin (FR); Blaise, Christian, 94160 Saint Mande (FR); Radisson, Xavier, 75014 Paris (FR)
(74) Mandataire: Bourdeau, Françoise

(57) **Abrégé**

L'invention a pour objet une composition cosmétique de défrisage des fibres kératiniques prête à l'emploi, le milieu cosmétiquement acceptable, contenant, en tant qu'agent actif de défrisage, une multiguanidine n'appartenant pas à la famille des hydroxydes. Elle vise également un kit contenant des compartiments à mettre en contact pour former la composition prête à l'emploi, ainsi qu'un procédé mettant en oeuvre cette composition.

## Description

L'invention a pour objet une composition cosmétique de défrisage des fibres kératiniques prête à l'emploi contenant, en tant qu'agent actif de défrisage, une multiguanidine n'appartenant pas à la famille des hydroxydes. Elle vise également un kit contenant des compartiments à mettre en contact pour former la composition prête à l'emploi, ainsi qu'un procédé mettant en oeuvre cette composition.

Le terme *"multiguanidine n'appartenant pas à la famille des hydroxydes"* englobe les multiguanidines et leurs sels organiques ou minéraux ne contenant pas dans leur formule chimique d'ions hydroxydes.

Par *"fibres kératiniques",* on entend, selon l'invention, des fibres d'origine humaine ou animale telles que les cheveux, les poils, les cils, la laine, l'angora, le cachemire ou la fourrure. Bien que l'invention ne soit pas limitée à des fibres kératiniques particulières, il sera néanmoins fait plus particulièrement référence aux cheveux.

Le terme *"défrisage"* englobe, selon l'invention, le défrisage, le lissage ou le décrêpage de cheveux caucasiens, asiatiques, nord-africains ou africains.

Le terme *"multiguanidine n'appartenant pas à la famille des hydroxydes"* désigne tout composé organique contenant dans sa formule au moins 2 fois le groupe suivant :
un atome de carbone doublement lié à un autre atome d'azote et simplement lié à deux autre atomes d'azote
et ne contenant pas dans sa formule chimique d'ions hydroxydes.

Avantageusement, selon l'invention, les multiguanidines contiennent au moins 2 fois le groupe guanidine, et de préférence 2 ou 3 fois.

Dans ce texte, l'expression *"compris entre x % et y* %" signifie allant de x à y %, les bornes x et y étant comprises.

Deux techniques sont utilisées pour obtenir une déformation permanente des cheveux. Elles sont basées sur une rupture des liaisons disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée. Cette technique permet indifféremment de réaliser, soit l'ondulation des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ■ La première réaction consiste en une béta-élimination sur la cystine provoquée par un ion hydroxyde, conduisant à la rupture de cette liaison et à la formation de déhydro-alanine. 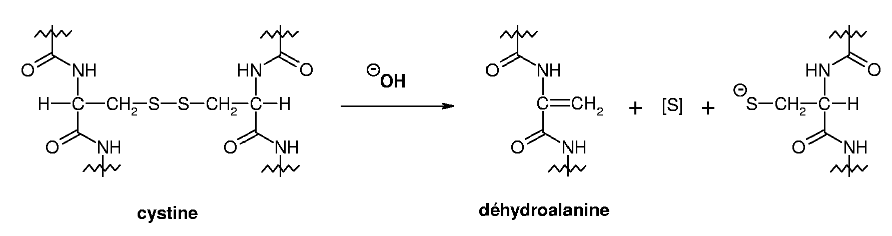
   ■ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

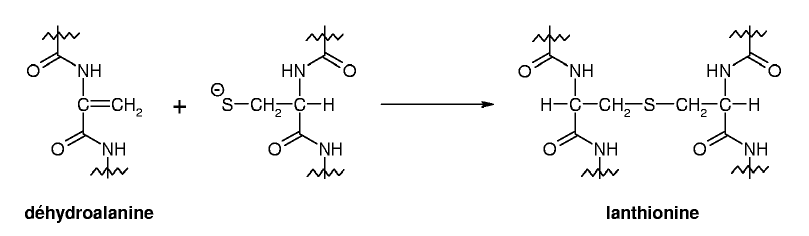

Par rapport à la première technique mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente (" hair waving "). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.

Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée "no-base", par opposition à l'appellation ci-dessus. Cette technologie " no-base " est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais " lye relaxers" et celles n'en contenant pas sont dénommées " no-lye relaxers ".

Les principales formules défrisantes dites "no-lye" mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium (" lye ") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas utilisées pour la permanente (" hair waving ") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour le défrisage de compositions moins agressives pour le cheveu et la peau.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, aussi bien pour la mise en forme que pour le défrisage et le lissage.

Pour remplacer les hydroxydes de sodium ou de guanidinium et pour améliorer la tolérance cutanée, il a été proposé, dans le brevet US4530830, d'utiliser une composition à base d'hydroxydes d'ammoniums quaternaires. Toutefois, ces compositions n'ont pas donné complète satisfaction, ni en terme de défrisage, ni en terme de cosmétique.

De façon plus générale, de nombreuses publications décrivent l'utilisation conjointe d'hydroxydes, servant d'actif de lanthionisation, avec certains additifs servant généralement à protéger les cheveux.

En effet, sans utiliser de nouveaux actifs de lanthionisation, les améliorations proposées concernent principalement l'utilisation d'additifs pour diminuer les dommages causés aux cheveux par les hydroxydes. On citera, à titre d'exemple:
- la demande WO2002/003937, qui décrit une composition contenant des monosaccharides en C3-C5,
- la demande WO2001/064171, qui décrit une composition contenant des agents complexants,
- le brevet US5641477, qui décrit une composition contenant un hydrolysat d'amidon hydrogéné,
- la demande WO02085317, qui décrit une composition contenant des nucléophiles organiques qui réagissent lors de la deuxième étape avec la déhydro-alanine formée avec des hydroxydes, pour conduire à de nouveaux pontages,
- le brevet US 5 679 327, qui décrit une composition de défrisage contenant nécessairement trois constituants actifs pour le défrisage, à savoir, un hydroxyde alcalin, un hydroxyde alcalino-terreux et une base organique azotée, chacun des constituants étant présent, dans la composition, dans une proportion qui serait insuffisante pour effectuer le défrisage s'il était utilisé à cette même concentration sans les deux autres actifs. En d'autres termes, ce brevet décrit une synergie entre trois constituants, ceux-ci conduisant ensemble au défrisage des fibres kératiniques.
Si toutes ces propositions conduisent à des améliorations plus ou moins marquées, elles ne permettent pas de diminuer de façon suffisante les dégâts liés à la causticité même des hydroxydes.

En rapport avec l'utilisation des hydroxydes pour le défrisage, il a aussi été divulgué, dans le brevet US 4 524 787, une composition prête à l'emploi réalisée à partir d'une partie "activateur" quasiment anhydre comprenant une base organique dans un solvant du type alcane polyhydroxylé et d'une partie aqueuse comprenant au moins 20 % d'eau. Néanmoins, ce dernier type de composition ne donne pas encore satisfaction, ni en terme de qualité de défrisage, ni en terme de propriétés mécaniques et cosmétiques des cheveux ayant subi ce traitement.

Comme indiqué précédemment, l'utilisation d'agents réducteurs conduit à une durabilité médiocre pour le défrisage ou le décrêpage et l'emploi d'hydroxydes, de part leur causticité, limite leur utilisation au domaine du défrisage.

Après d'importantes études, on vient maintenant de découvrir, de façon tout à fait surprenante et inattendue, que l'on pouvait effectuer la première étape du procédé de lanthionisation avec des multiguanidines n'appartenant pas à la familles des hydroxydes. On obtient ainsi d'excellents résultats en terme de défrisage, de propriétés cosmétiques et mécaniques des cheveux.

La présente invention a pour objet une composition cosmétique comprenant, dans un milieu cosmétiquement acceptable, au moins une multiguanidine n'appartenant pas à la famille des hydroxydes, le milieu cosmétiquement acceptable et la multiguanidine étant choisis de façon telle que la multiguanidine n'appartenant pas à la famille des hydroxydes réagisse sur les cystines des fibres kératiniques, par une réaction de bêta-élimination produisant de la déhydro-alanine et conduisant à la formation de lanthionine, pour défriser les fibres kératiniques en moins de 60 minutes

Avantageusement, la durée du défrisage est inférieure à 40 minutes, et plus avantageusement inférieure à 30 minutes.

Par *"multiguanidines n'appartenant pas à la familles de hydroxydes",* utilisables à titre d'actifs de béta-élimination conduisant à la lanthionisation, on entend tout composé organique contenant dans sa formule au moins 2 fois le groupe suivant :
un atome de carbone doublement lié à un autre atome d'azote et simplement lié à deux autre atomes d'azote
ne contenant pas dans sa formule d'ions hydroxydes et capable d'accepter un proton.

Les multiguanidines selon l'invention sont principalement choisies dans le groupe des composés répondant à la formule générale(I) suivante : 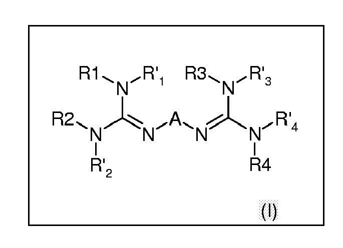 dans laquelle :
- R1,R'1,R2,R'2,R3,R'3,R4,R'4, identiques ou différents représentent un radical choisi parmi :
   - un atome d'hydrogène
   - un groupement alkyle en C1 à C6, saturé ou insaturé, linéaire tel que par exemple méthyle ou éthyle, ou ramifié tel que par exemple isopropyle ou tertbutyle, ou encore cyclique tel que par exemple cyclopentyle ou cyclohexyle, ledit groupement alkyle étant éventuellement substitué par le radical suivant : 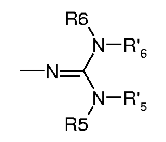 dans lequel R5,R'5,R6 et R'6 ont les mêmes significations que les radicaux R1 à R'4 désignés ci-dessus
- R1et R2 et/ou R3 et R4 peuvent également former ensemble un radical divalent (CH2)2 ou (CH2)3 ou CH=CH.
- A représente un radical hydrocarboné divalent en C2 à C12, linéaire ou cyclique, saturé ou insaturé , pouvant être éventuellement interrompu par un ou plusieurs groupes tels que imino,carboxamido, sulfoxyde, sulfone, et/ou par :
   - un ou plusieurs hétéoatomes choisis parmi le soufre, l'oxygène, l'azote et le silicium et éventuelllement substitué par :
   - un radical alkyle en C1 à C4 linéaire ou ramifié éventuellement interrompu par un ou plusieurs hétéroatomes tels que définis ci-dessus et éventuellement substitué par le radical suivant : 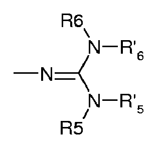 dans lequel R5,R'5,R6 et R'6 ont les mêmes significations que les radicaux R1 à R'4 désignés ci-dessus.

Les composés de formule générale (I) sont préparés selon les schémas de synthèse suivants:
Schéma 1 :
   Le sel de Vilsmeyer **1** (préparé par action du phosgène ou d'un substitut tel que le chorure d'oxalyle ou l'oxychlorure de phosphore sur la tétraalkyl urée) est mis en réaction avec la diamine **2** (ou triamine **3)** dans un solvant aprotique polaire tel que l'acétonitrile en présence d'une base organique tertiaire telle que par exemple la triéthylamine pour conduire aux composés de formule (I). 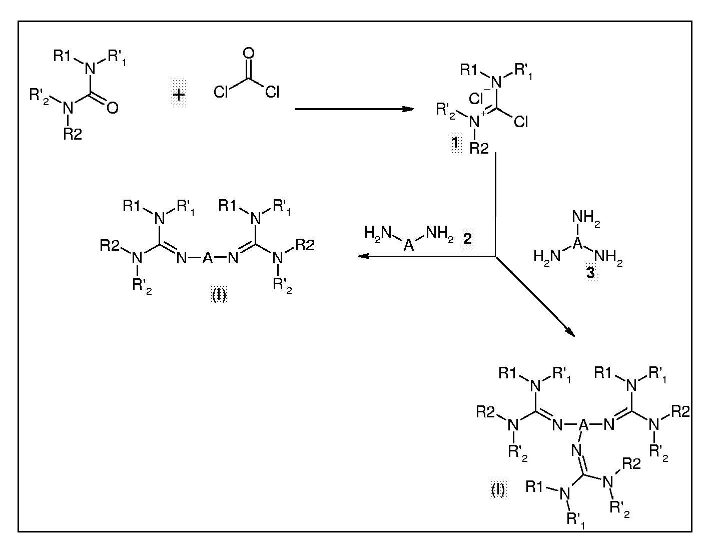
Schéma 2 :
   Le dérivé dihalogéné **4** est mis en réaction avec un large excés de la tétraalkylguanidine **5** à une température proche de 100°C. L'excés de tétraalkylguanidine est éliminé par évaporation sous pression réduite et le résidu est traité par une base telle que par exemple l'éthylate de sodium dans l'éthanol.
   Le sel est éliminé par filtration puis le dérivé de formule (I) attendu est ensuite obtenu et purifié généralement par distillation sous pression réduite.

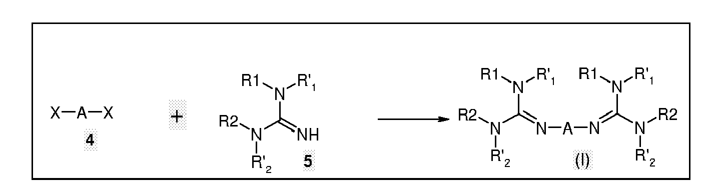

On pourra se reporter utilement aux modes opératoires décrits dans les références suivantes :
-1)- Z.anorg. Allg.Chem.2000,.626,1583-1590
-2) Inorg.Chem.2001,40,6964-6971
-3) J.Org.Chem. 2003,68,8790-8797
-4) J.Chem.Soc. ; Dalton Trans., 2000,3473-3479

Les composés de formule (I) préférés sont les suivants:
- N,N'-bis(1,3-dimethyl-2-imidazolidinylidene)-1,2-benzenediamine (RN: 774610-65-0)
- N-[bis(dimethylamino)methylene]-3-(dimethylamino)-5-imino-2-methyl-2,4,6,19-Tetraazaeicosan-20-imidamide (RN : 791543-84-5)
- N,N'-bis(tetrahydro-1,3-dimethyl-2(1H)-pyrimidinylidene)-1,3-propanediamine (RN :752232-69-2)
- 1,1',1'',1'''-[1propanediylbis(nitrilomethanetetrayl)]tetrakis-piperidine (RN : 752232-68-1)
- Poly(oxy-1,2-ethanediyl), α-[2-[(1,3-dimethyl-2-imidazolidinylidene)amino]ethyl]-ω-[2-[(1,3-dimethyl-2-imidazolidinylidene)amino]ethoxy] (RN : 742679-23-8)
- N",N""'-1,2-ethanediylbis[N,N,N',N'-tetrakis(1-methylethyl)-guanidine (RN : 676488-06-5)
- N,N'-bis(1,3-dihydro-1,3-dimethyl-2H-imidazol-2-ylidene)-1,8-naphtalenediamine (RN : 634192-99-7)
- N,N'-bis(1,3-dimethyl-2-imidazolidinylidene)- 1,8-Naphthalenediamine (RN : 501931-38-0)
- N'',N'''''-1,8-biphenylenediylbis[N,N,N',N'-tetramethyl-guanidine] (RN : 495408-66-7)
- N'',N'''''-benzo[c]phenanthrene-1,12-diylbis[N,N,N',N'-tetramethyl-guanidine] (RN : 495408-58-7)
- N'',N'''''-4,5-phenanthrenediylbis[N,N,N',N'-tetramethyl-guanidine] (RN : 495408-50-9)
- N'',N'''''-(9,10-dihydro-4,5-phenanthrenediyl)bis[N,N,N',N'-tetramethylguanidine] (RN : 495408-44-1)
- N'',N'''''-1,2-phenylenebis[N,N,N',N'-tetramethyl-guanidine] (RN : 495408-35-0)
- N'',N'''''-1,8-naphthalenediylbis[N,N,N',N'-tetramethyl-guanidine] hydrochloride (RN : 443892-20-4)
- N",N'''''-9H-fluorene-4,5-diylbis[N,N,N',N'-tetramethyl-guanidine] (RN : 443892-12-4)
- N'-(tetrahydro-1,3-dimethyl-2(1H)-pyrimidinylidene)-N,N-bis[2-[(tetrahydro-1,3-dimethyl-2(1H)-pyrimidinylidene)amino]ethyl]-1,2-ethanediamine (RN : 395640-62-7)
- N",N""',N""""-(nitrilotri-2,1-ethanediyl)tris[N,N,N',N'-tetramethyl-guanidine] (RN : 368866-05-1)
- N",N""'-[2-[[[bis(dimethylamino)methylene]amino]methyl]-2-methyl-1,3-propanediyl]bis[N,N,N',N'-tetramethyl-guanidine] (RN : 289474-30-2)
- N",N""'-(2,2-dimethyl-1,3-propanediyl)bis[N,N,N',N'-tetramethyl-guanidine] (RN : 289474-29-9)
- N",N""'-1,3-propanediylbis[N,N,N',N'-tetramethyl-guanidine](RN :289474-28-8)
- N",N""'-1,2-ethanediylbis[N,N,N',N'-tetramethyl guanidine] (RN : 289474-25-5)
- N,N'-bis(1,3-dihydro-1,3-dimethyl-2H-imidazol-2-ylidene)-1,2-Ethanediamine (RN : 216873-26-6)
- N,N"'-1,2-ethanediylbis[N',N',N",N"-diethyl-guanidine] (RN : 211869-99-7)
- N",N""'-[(methylimino)di-3,1-propanediyl]bis[N,N,N',N'-tetramethylguanidine] (RN : 196405-86-4)
- N'',N'''''-[oxybis(2,1-ethanediyloxy-3,1-propanediyl)]bis [N,N,N',N'-tetramethylguanidine] (RN : 190442-53-6)
- N'',N'''''-[[1,1,3,3-tetramethyl-1,3-disiloxanediyl]di-3,1-propanediyl]bis[N,N,N',N'-tetramethylguanidine] (RN : 175989-14-7)
- N'',N'''''-[[1,3-dimethyl-1,3-bis[(trimethylsilyl)oxy]-1,3-disiloxanediyl]di-3,1-propanediyl]bis[N,N,N',N'-tetramethyl-guanidine] (RN : 172283-48-6)
- N,N"-1,6-hexanediylbis[N'-[bis(dimethylamino)methylene urea (RN : 157362-45-3)
- [bis(dimethylamino)methylene][3[[[[[bis(dimethylamino)methyl ene]amino]carbonyl]amino]methyl]-3,5,5-trimethylcyclohexyl]-urea (RN: 157362-44-2)
- N'',N'''''-[1,1'-biphenyl]-2,2'-diylbis[N,N,N',N'-tetramethyl-guanidine] (RN: 144576-63-6)
- 4,4'-sulfonylbis[N-(1,3-dimethyl-2-imidazolidinylidene)-benzenamine (RN : 129346-76-5)
- N,N'-bis(1,3-dimethyl-2-imidazolidinylidene),2,8-dibenzothiophenediamine-5,5-dioxide (RN : 128169-35-7)
- N,N'-bis(1,3-dimethyl-2-imidazolidinylidene)-4,6-Dibenzothiophenediamine -5,5-dioxide (RN : 127330-56-7)
- N,N'-bis(1,3-dimethyl-2-imidazolidinylidene)- 1,2-Ethanediamine (RN : 126620-51-7)
- N",N""'-1,3-phenylenebis[N,N,N',N'-tetramethyl-guanidine] (RN : 121648-84-8)
- N,N'-bis[bis(dimethylamino)methylene]-butanediamine (RN : 114491-72-4)
- N'',N'''''-1,4-phenylenebis[N,N,N',N'-tetramethyl -guanidine] (RN: 113551-45-4)
- N'',N'''''-[[1,3-dimethyl-1-[(pentamethyldisiloxanyl)oxy]-3-[(trimethylsilyl)oxy]- 1,3-disiloxanediyl]di-3,1-propanediyl]bis[N,N,N',N'-tetramethylguanidine]
   (RN : 109956-31-2)
- N,N"-bis[(dimethylamino)(dipentylamino)methylene]-1,4-Piperazinedicarboximidamide (RN : 97983-93-2)
- ,N,N"-bis[(dimethylamino)(hexylpropylamino)methylene]-1,4-Piperazinedicarboximidamide (RN : 97983-92-1)
- N,N"-bis[(hexylmethylamino)(methylpropylamino)methylene]-1,4-Piperazinedicarboximidamide (RN : 97983-91-0)
- N,N"-bis[(butylmethylamino)(hexylmethylamino)methylene]-1,4-Piperazinedicarboximidamide (RN : 97983-90-9)
- N,N"-bis[(dimethylamino)(heptylmethylamino)methylene]-1,4-Piperazinedicarboximidamide (RN : 97963-91-2)
- N",N""'-[(1,1,3,3-tetramethoxy-1,3-disiloxanediyl)di-3,1-propanediyl]bis[N,N,N',N'-tetramethyl-guanidine] (RN : 69755-28-8)
- N'',N'''''-1,6-hexanediylbis[N,N,N',N'-tetramethyl-guanidine] (RN : 64933-93-3)
- N'',N'''''-(methylenedi-4,1-phenylene)bis[N,N,N',N'-tetramethyl-guanidine] (RN : 57414-23-0)
- N,N"-(methylphenylene)bis[N'-[bis(dimethylamino)methylene]-urea (RN : 39529-23-2)
- 2,2'-(sulfonyldiethylene)bis[1,1,3,3-tetramethyl guanidine] (RN : 13998-89-5)

Les composés de formule générale (I) plus particulièrement préférés sont les suivants :
- N-[bis(dimethylamino)methylene]-3-(dimethylamino)-5-imino-2-methyl-2,4,6,19-Tetraazaeicosan-20-imidamide (RN : 791543-84-5)
- N,N'-bis(tetrahydro-1,3-dimethyl-2(1H)-pyrimidinylidene)-1,3-propanediamine(RN :752232-69-2)
- Poly(oxy-1,2-ethanediyl), α-[2-[(1,3-dimethyl-2-imidazolidinylidene)amino]ethyl]-ω-[2-[(1,3-dimethyl-2-imidazolidinylidene)amino]ethoxy] (RN : 742679-23-8)
- N",N""'-1,2-ethanediylbis[N,N,N',N'-tetrakis(1-methylethyl)-guanidine (RN : 676488-06-5)
- N,N'-bis(1,3-dihydro-1,3-dimethyl-2H-imidazol-2-ylidene)-1,8-naphtalenediamine (RN : 634192-99-7)
- N,N'-bis(1,3-dimethyl-2-imidazolidinylidene)- 1,8-Naphthalenediamine (RN : 501931-38-0)
- N",N""'-1,2-phenylenebis[N,N,N',N'-tetramethyl-guanidine] (RN : 495408-35-0)
- N",N""'-1,8-naphthalenediylbis[N,N,N',N'-tetramethyl-guanidine] hydrochloride (RN : 443892-20-4)
- N",N""',N""""-(nitrilotri-2,1-ethanediyl)tris[N,N,N',N'-tetramethyl-guanidine] (RN : 368866-05-1)
- N",N""'-[2-[[[bis(dimethylamino)methylene]amino]methyl]-2-methyl-1,3-propanediyl]bis[N,N,N',N'-tetramethyl-guanidine] (RN : 289474-30-2)
- N",N""'-(2,2-dimethyl-1,3-propanediyl)bis[N,N,N',N'-tetramethyl-guanidine] (RN : 289474-29-9)
- N",N'''''-1,3-propanediylbis[N,N,N',N'-tetramethyl-guanidine](RN :289474-28-8)
- N,N'-bis(1,3-dihydro-1,3-dimethyl-2H-imidazol-2-ylidene)-1,2-Ethanediamine (RN : 216873-26-6)
- N,N'''-1,2-ethanediylbis[N',N',N",N"-diethyl-guanidine] (RN : 211869-99-7)
- N",N'''''-[(methylimino)di-3,1-propanediyl]bis[N,N,N',N'-tetramethylguanidine] (RN : 196405-86-4)
- N'',N'''''-[oxybis(2,1-ethanediyloxy-3,1-propanediyl)]bis[N,N,N',N'-tetramethylguanidine] (RN : 190442-53-6)
- N'',N'''''-[[1,1,3,3-tetramethyl-1,3-disiloxanediyl]di-3,1-propanediyl]bis[N,N,N',N'-tetramethylguanidine] (RN : 175989-14-7)
- N,N''-1,6-hexanediylbis[N'-[bis(dimethylamino)methylene urea (RN : 157362-45-3)
- N",N""'-[1,1'-biphenyl]-2,2'-diylbis[N,N,N',N'-tetramethyl-guanidine] (RN: 144576-63-6)
- N,N'-bis(1,3-dimethyl-2-imidazolidinylidene)- 1,2-Ethanediamine (RN : 126620-51-7)
- N'',N'''''-1,4-phenylenebis[N,N,N',N'-tetramethyl -guanidine] (RN: 113551-45-4)
- N'',N'''''-[(1,1,3,3-tetramethoxy-1,3-disiloxanediyl)di-3,1-propanediyl]bis[N,N,N',N'-tetramethyl-guanidine] (RN : 69755-28-8)
- N'',N'''''-1,6-hexanediylbis[N,N,N',N'-tetramethyl-guanidine] (RN : 64933-93-3)
- 2,2'-(sulfonyldiethylene)bis[1,1,3,3-tetramethyl guanidine] (RN : 13998-89-5)

L'invention concerne également les nouveaux composés de formule générale (II) :

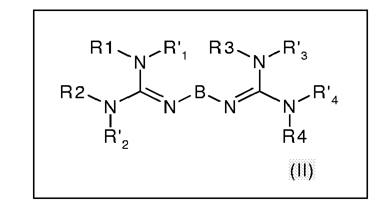

Dans laquelle :
- R1,R'1,R2,R'2,R3,R'3,R4,R'4, identiques ou différents représentent un groupement choisi parmi :
   - un atome d'hydrogène
   - un radical méthyle, éthyle, propyle ou isopropyle
      R1et R2 et/ou R3 et R4 peuvent également former ensemble un radical divalent (CH2)2 ou (CH2)3 ou CH=CH.
- B représente un radical choisi parmi : 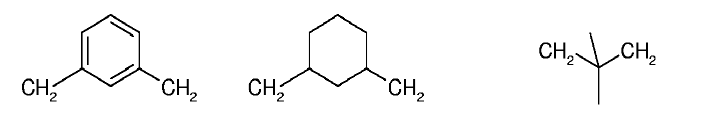 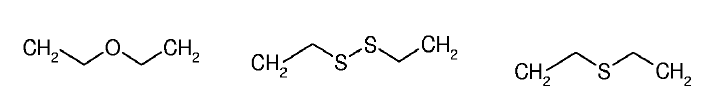 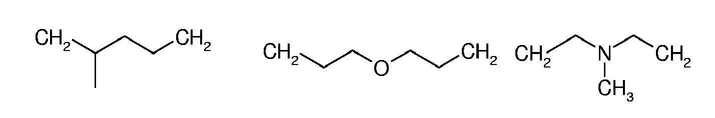 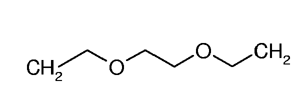
- Lorsque R'1 =R'2= R'3= R'4 = CH3 et lorsque R1/R2 et R3/R4 forment respectivement et simultanément le radical divalent (CH2)2 ;
   B représente également un radical divalent (CH2)2 ou (CH2)5 ou (CH2)6 ou un radical : 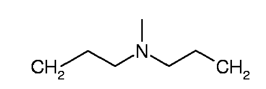
- Lorsque R'1 =R'2= R'3= R'4 = CH3 et lorsque R1/R2 et R3/R4 forment respectivement et simultanément le radical divalent (CH2)3;
   B représente (CH2)2 ou (CH2)3 ou (CH2)4 ou (CH2)5 ou (CH2)6 ou un radical : 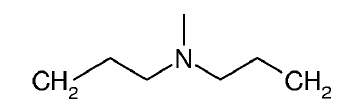
- Lorsque R'1 =R'2= R'3= R'4 = CH3 et lorsque R1/R2 et R3/R4 forment respectivement et simultanément le radical divalent CH=CH :
   B représente (CH2)3 ou (CH2)4 ou (CH2)5 ou (CH2)6 ou un radical : 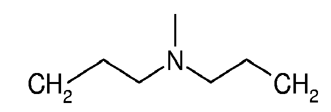
- Lorsque R1,R'1,R2,R'2,R3,R'3,R4,R'4 représentent un radical ethyle ou isopropyle, B représente un radical (CH2)3 ou (CH2)4 ou (CH2)5 ou (CH2)6 ou un radical : 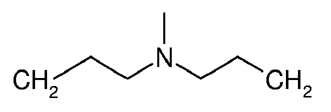
- Lorsque R1,R'1,R2,R'2,R3,R'3,R4,R'4 représentent un radical méthyle, B représente un radical (CH2)4 ou (CH2)5.

Les composés préférés de formule générale (II) sont :
- N,N,N',N'-Tetraisopropyl-N"-[3-(N',N',N",N"-tetraisopropyl-guanidino)-propyl]-guanidine
- N,N,N',N'-Tetraisopropyl-N"-[4-(N',N',N",N"-tetraisopropyl-guanidino)-butyl]-guanidine
- N,N,N',N'-Tetraisopropyl-N"-[5-(N',N',N",N"-tetraisopropyl-guanidino)-pentyl]-guanidine
- N,N,N',N'-Tetraisopropyl-N''-[6-(N',N',N'',N''-tetraisopropyl-guanidino)-hexyl]-guanidine
- N,N,N',N'-Tetraisopropyl-N''-(3-{methyl-[3-(N',N',N'',N''-tetraisopropylguanidino)-propyl]-amino }-propyl)-guanidine
- N,N,N',N'-Tetraethyl-N"-[3-(N',N',N",N"-tetraethyl-guanidino)-propyl]-guanidine
- N,N,N',N'-Tetraethyl-N''-[4-(N',N',N'',N''-tetraethyl-guanidino)-butyl]-guanidine
- N,N,N',N'-Tetraethyl-N"-[5-(N',N',N",N"-tetraethyl-guanidino)-pentyl]-guanidine
- N,N,N',N'-Tetraethyl-N''-[6-(N',N',N",N''-tetraethyl-guanidino)-hexyl]-guanidine
- N,N,N',N'-Tetraethyl-N"-(3-{methyl-[3-(N',N',N'',N''-tetraisopropyl-guanidino)-propyl]-amino}-propyl)-guanidine
- N,N'-Bis-(1,3-dimethyl-imidazolidin-2-ylidene)-butane-1,4-diamine
- N,N'-Bis-(1,3-dimethyl-imidazolidin-2-ylidene)-pentane-1,5-diamine
- N,N'-Bis-(1,3-dimethyl-imidazolidin-2-ylidene)-hexane-1,6-diamine
- N-(1,3-Dimethyl-imidazolidin-2-ylidene)-N'-[3-(1,3-dimethyl-imidazolidin-2-ylideneamino)-propyl]-N'-methyl-propane-1,3-diamine
- N,N'-Bis-(1,3-dimethyl-1,3-dihydro-imidazol-2-ylidene)-propane-1,3-diamine
- N,N'-Bis-(1,3-dimethyl-1,3-dihydro-imidazol-2-ylidene)-butane-1,4-diamine
- N,N'-Bis-(1,3-dimethyl-1,3-dihydro-imidazol-2-ylidene)-pentane-1,5-diamine
- N,N'-Bis-(1,3-dimethyl-1,3-dihydro-imidazol-2-ylidene)-hexane-1,6-diamine
- N-(1,3-Dimethyl-1,3-dihydro-imidazol-2-ylidene)-N'-[3-(1,3-dimethyl-1,3-dihydro-imidazol-2-ylideneamino)-propyl]-N'-methyl-propane-1,3-diamine
- N,N'-Bis-(1,3-dimethyl-tetrahydro-pyrimidin-2-ylidene)-ethane-1,2-diamine
- N,N'-Bis-(1,3-dimethyl-tetrahydro-pyrimidin-2-ylidene)-propane-1,3-diamine
- N,N'-Bis-(1,3-dimethyl-tetrahydro-pyrimidin-2-ylidene)-butane-1,4-diamine
- N,N'-Bis-(1,3-dimethyl-tetrahydro-pyrimidin-2-ylidene)-pentane-1,5-diamine
- N,N'-Bis-(1,3-dimethyl-tetrahydro-pyrimidin-2-ylidene)-hexane-1,6-diamine
- N-(1,3-Dimethyl-tetrahydro-pyrimidin-2-ylidene)-N'-[3-(1,3-dimethyltetrahydro-pyrimidin-2-ylideneamino)-propyl]-N'-methyl-propane-1,3-diamine
- N,N,N',N'-Tetramethyl-N"-[4-(N',N',N",N"-tetramethyl-guanidino)-butyl]-guanidine
- N,N,N',N'-Tetramethyl-N"-[5-(N',N',N",N"-tetramethyl-guanidino)-pentyl]-guanidine
- N,N,N',N'-Tetramethyl-N"-[3-(N',N',N",N"-tetramethyl-guanidinomethyl)-cyclohexylmethyl]-guanidine
- N,N,N',N'-Tetraethyl-N"-[3-(N',N',N",N"-tetraethyl-guanidinomethyl)-cyclohexylmethyl]-guanidine
- N,N,N',N'-Tetrapropyl-N"-[3-(N',N',N",N"-tetrapropyl-guanidinomethyl)-cyclohexylmethyl]-guanidine
- N,N,N',N'-Tetraisopropyll-N"-[3-(N',N',N",N"-tetraisopropyl-guanidinomethyl)-cyclohexylmethyl]-guanidine
- N,N,N',N'-Tetraethyl-N"-[3-(N',N',N",N"-tetraethyl-guanidinomethyl)-benzyl]-guanidine
- N,N,N',N'-Tetrapropyll-N"-[3-(N',N',N",N"-tetrapropyl-guanidinomethyl)-benzyl]-guanidine
- N,N,N',N'-Tetraisopropyl-N"-[3-(N',N',N",N"-tetraisopropyl-guanidinomethyl)-benzyl]-guanidine
- N"-[2,2-Dimethyl-3-(N',N',N",N"-tetraethyl-guanidino)-propyl]-N,N,N',N'-tetraethyl-guanidine
- N"-[2,2-Dimethyl-3-(N',N',N",N"-tetrapropyl-guanidino)-propyl]-N,N,N',N'-tetrapropyl-guanidine
- N"-[2,2-Dimethyl-3-(N',N',N",N"-tetraisopropyl-guanidino)-propyl]-N,N,N',N'-tetraisopropyl-guanidine
- N,N,N',N'-Tetramethyl-N"-{2-[2-(N',N',N",N"-tetramethyl-guanidino)-ethoxy]-ethyl}-guanidine
- N,N,N',N'-Tetraethyl-N"-{2-[2-(N',N',N",N"-tetraethyl-guanidino)-ethoxy]-ethyl}-guanidine
- N,N,N',N'-Tetrapropyl-N"-{2-[2-(N',N',N",N"-tetrapropyl-guanidino)-ethoxy]-ethyl}-guanidine
- N,N,N',N'-Tetraisopropyl-N"-{2-[2-(N',N',N",N"-tetraisopropyl-guanidino)-ethoxy]-ethyl}-guanidine
- N,N,N',N'-Tetramethyl-N"-{2-[2-(N',N',N",N"-tetramethyl-guanidino)-ethylsulfanyl]-ethyl}-guanidine
- N,N,N',N'-Tetraethyl-N"-{2-[2-(N',N',N",N"-tetraethyl-guanidino)-ethylsulfanyl]-ethyl}-guanidine
- N,N,N',N'-Tetrapropyl-N"-{2-[2-(N',N',N",N"-tetrapropyl-guanidino)-ethylsulfanyl]-ethyl}-guanidine
- N,N,N',N'-Tetraisopropyl-N"-{2-[2-(N',N',N",N"-tetraisopropyl-guanidino)-ethylsulfanyl]-ethyl}-guanidine
- N,N,N',N'-Tetramethyl-N"-{2-[2-(N',N',N",N"-tetramethyl-guanidino)-ethyldisulfanyl]-ethyl}-guanidine
- N,N,N',N'-Tetraethyl-N''-{2-[2-(N',N',N'',N''-tetraethyl-guanidino)-ethyldisulfanyl]-ethyl}-guanidine
- N,N,N',N'-Tetrapropyl-N''-{2-[2-(N',N',N'',N''-tetrapropyl-guanidino)-ethyldisulfanyl]-ethyl}-guanidine
- N,N,N',N'-Tetraisopropyl-N''-{2-[2-(N',N',N'',N''-tetraisopropyl-guanidino)-ethyldisulfanyl]-ethyl}-guanidine
- N,N,N',N'-Tetramethyl-N"-[2-methyl-5-(N',N',N",N"-tetramethyl-guanidino)-pentyl]-guanidine
- N,N,N',N'-Tetraethyl-N"-[2-methyl-5-(N',N',N",N"-tetraethyl-guanidino)-pentyl]-guanidine
- N,N,N',N'-Tetrapropyl-N"-[2-methyl-5-(N',N',N",N"-tetrapropyl-guanidino)-pentyl]-guanidine
- N,N,N',N'-Tetraisopropyl-N"-[2-methyl-5-(N',N',N",N"-tetraisopropylguanidino)-pentyl]-guanidine
- N,N,N',N'-Tetramethyl-N''-{3-[3-(N',N',N'',N''-tetramethyl-guanidino)-propoxy]-propyl}-guanidine
- N,N,N',N'-Tetraethyl-N''-{3-[3-(N',N',N'',N''-tetraethyl-guanidino)-propoxy]-propyl}-guanidine
- N,N,N',N'-Tetrapropyl-N''-{3-[3-(N',N',N'',N''-tetrapropyl-guanidino)-propoxy]-propyl}-guanidine
- N,N,N',N'-Tetraisopropyl-N''-{3-[3-(N',N',N'',N'-tetraisopropyl-guanidino)-propoxy]-propyl}-guanidine
- N,N,N',N'-Tetramethyl-N''-(2-{methyl-[2-(N',N',N'',N''-tetramethyl-guanidino)-ethyl]-amino }-ethyl)-guanidine
- N,N,N',N'-Tetraethyl-N''-(2-{methyl-[2-(N',N',N'',N''-tetraethyl-guanidino)-ethyl]-amino}-ethyl)-guanidine
- N,N,N',N'-Tetrapropyl-N''-(2-{methyl-[2-(N',N',N'',N''-tetrapropyl-guanidino)-ethyl]-amino }-ethyl)-guanidine
- N,N,N',N'-Tetraisopropyl-N''-(2-{methyl-[2-(N',N',N'',N''-tetraisopropylguanidino)-ethyl]-amino }-ethyl)-guanidine
- N,N,N',N'-Tetramethyl-N''-(2-{2-[2-(N',N',N'',N''-tetramethyl-guanidino)-ethoxy]-ethoxy}-ethyl)-guanidine
- N,N,N',N'-Tetraethyl-N''-(2-{2-[2-(N',N',N'',N''-tetraethyl-guanidino)-ethoxy]-ethoxy}-ethyl)-guanidine
- N,N,N',N'-Tetrapropyl-N''-(2-{2-[2-(N',N',N'',N''-tetrapropyl-guanidino)-ethoxy]-ethoxy}-ethyl)-guanidine
- N,N,N',N'-Tetraisopropyl-N''-(2-{2-[2-(N',N',N'',N''-tetraisopropyl-guanidino)-ethoxy]-ethoxy}-ethyl)-guanidine
- 2-{3-[(1,3-Dimethyl-imidazolidin-2-ylideneamino)-methyl]-cyclohexylmethylimino }-1,3-dimethyl-imidazolidine
- 2-{3-[(1,3-Dimethyl-imidazolidin-2-ylideneamino)-methyl]-benzylimino}-1,3-dimethyl-imidazolidine
- N,N'-Bis-(1,3-dimethyl-imidazolidin-2-ylidene)-2,2-dimethyl-propane-1,3-diamine
- 2-{2-[2-(1,3-Dimethyl-imidazolidin-2-ylideneamino)-ethylsulfanyl]-ethylimino}-l,3-dimethyl-imidazolidine
- 2-{2-[2-(1,3-Dimethyl-imidazolidin-2-ylideneamino)-ethyldisulfanyl]-ethylimino }-1,3-dimethyl-imidazolidine
- N1,N5-Bis-(1,3-dimethyl-imidazolidin-2-ylidene)-2-methyl-pentane-1,5-diamine
- N-(1,3-Dimethyl-imidazolidin-2-ylidene)-N'-[2-(1,3-dimethyl-imidazolidin-2-ylideneamino)-ethyl]-N'-methyl-ethane-1,2-diamine
- N1,N5-Bis-(1,3-dimethyl-1,3-dihydro-imidazol-2-ylidene)-2-methyl-pentane-1,5-diamine
- N-(1,3-Dimethyl-1,3-dihydro-imidazol-2-ylidene)-N'-[2-(1,3-dimethyl-1,3-dihydro-imidazol-2-ylideneamino)-ethyl]-N'-methyl-ethane-1,2-diamine
- 2-{3-[(1,3-Dimethyl-1,3-dihydro-imidazol-2-ylideneamino)-methyl]-cyclohexylmethylimino}-l,3-dimethyl-2,3-dihydro-1H-imidazole
- N,N'-Bis-(1,3-dimethyl-1,3-dihydro-imidazol-2-ylidene)-2,2-dimethyl-propane-1,3-diamine
- 2-{3-[(1,3-Dimethyl-1,3-dihydro-imidazol-2-ylideneamino)-methyl]-benzylimino }-1,3-dimethyl-2,3-dihydro-1H-imidazole
- 2-{2-[2-(1,3-Dimethyl-1,3-dihydro-imidazol-2-ylideneamino)-ethyldisulfanyl]-ethylimino}-1,3-dimethyl-2,3-dihydro-1H-imidazole
- 2-{2-[2-(1,3-Dimethyl-1,3-dihydro-imidazol-2-ylideneamino)-ethylsulfanyl]-ethylimino }-1,3-dimethyl-2,3-dihydro-1H-imidazole
- N1,N5-Bis-(1,3-dimethyl-tetrahydro-pyrimidin-2-ylidene)-2-methyl-pentane-1,5-diamine
- N-(1,3-Dimethyl-tetrahydro-pyrimidin-2-ylidene)-N'-[2-(1,3-dimethyltetrahydro-pyrimidin-2-ylideneamino)-ethyl]-N'-methyl-ethane-1,2-diamine
- 2-{3-[(1,3-Dimethyl-tetrahydro-pyrimidin-2-ylideneamino)-methyl]-cyclohexylmethylimino }-1,3-dimethyl-hexahydro-pyrimidine
- N,N'-Bis-(1,3-dimethyl-tetrahydro-pyrimidin-2-ylidene)-2,2-dimethyl-propane-1,3-diamine
- 2-{3-[(1,3-Dimethyl-tetrahydro-pyrimidin-2-ylideneamino)-methyl]-benzylimino }-1,3-dimethyl-hexahydro-pyrimidine
- 2-{2-[2-(1,3-Dimethyl-tetrahydro-pyrimidin-2-ylideneamino)-ethyldisulfanyl]-ethylimino }-1,3-dimethyl-hexahydro-pyrimidine
- 2-{2-[2-(1,3-Dimethyl-tetrahydro-pyrimidin-2-ylideneamino)-ethylsulfanyl]-ethylimino }-1,3-dimethyl-hexahydro-pyrimidine

Les composés particulièrement préférés de formule générale (II) sont:
- N,N,N',N'-Tetraisopropyl-N"-[3-(N',N',N",N"-tetraisopropyl-guanidino)-propyl]-guanidine
- N,N,N',N'-Tetraisopropyl-N"-[4-(N',N',N",N"-tetraisopropyl-guanidino)-butyl]-guanidine
- N,N,N',N'-Tetraethyl-N"-[3-(N',N',N",N"-tetraethyl-guanidino)-propyl]-guanidine
- N,N,N',N'-Tetraethyl-N"-[4-(N',N',N",N"-tetraethyl-guanidino)-butyl]-guanidine
- N,N'-Bis-(1,3-dimethyl-imidazolidin-2-ylidene)-butane-1,4-diamine
- N-(1,3-Dimethyl-imidazolidin-2-ylidene)-N'-[3-(1,3-dimethyl-imidazolidin-2-ylideneamino)-propyl]-N'-methyl-propane-1,3-diamine
- N,N'-Bis-(1,3-dimethyl-1,3-dihydro-imidazol-2-ylidene)-propane-1,3-diamine
- N,N'-Bis-(1,3-dimethyl-1,3-dihydro-imidazol-2-ylidene)-butane-1,4-diamine
- N-(1,3-Dimethyl-1,3-dihydro-imidazol-2-ylidene)-N'-[3-(1,3-dimethyl-1,3-dihydro-imidazol-2-ylideneamino)-propyl]-N'-methyl-propane-1,3-diamine
- N,N'-Bis-(1,3-dimethyl-tetrahydro-pyrimidin-2-ylidene)-ethane-1,2-diamine
- N,N'-Bis-(1,3-dimethyl-tetrahydro-pyrimidin-2-ylidene)-propane-1,3-diamine
- N,N,N',N'-Tetramethyl-N"-[4-(N',N',N",N"-tetramethyl-guanidino)-butyl]-guanidine
- N,N,N',N'-Tetramethyl-N"-[3-(N',N',N",N"-tetramethyl-guanidinomethyl)-cyclohexylmethyl]-guanidine
- N,N,N',N'-Tetraethyl-N"-[3-(N',N',N",N"-tetraethyl-guanidinomethyl)-benzyl]-guanidine
- N"-[2,2-Dimethyl-3-(N',N',N",N"-tetraethyl-guanidino)-propyl]-N,N,N',N'-tetraethyl-guanidine
- N,N,N',N'-Tetramethyl-N''-{2-[2-(N',N',N'',N''-tetramethyl-guanidino)-ethoxy]-ethyl}-guanidine
- N,N,N',N'-Tetramethyl-N''-{2-[2-(N',N',N'',N''-tetramethyl-guanidino)-ethylsulfanyl]-ethyl}-guanidine
- N,N,N',N'-Tetramethyl-N''-{2-[2-(N',N',N'',N''-tetramethyl-guanidino)-ethyldisulfanyl]-ethyl}-guanidine
- N,N,N',N'-Tetramethyl-N"-[2-methyl-5-(N',N',N",N"-tetramethyl-guanidino)-pentyl]-guanidine
- N,N,N',N'-Tetramethyl-N''-{3-[3-(N',N',N'',N''-tetramethyl-guanidino)-propoxy]-propyl}-guanidine
- N,N,N',N'-Tetramethyl-N"-(2-{methyl-[2-(N',N',N",N"-tetramethyl-guanidino)-ethyl]-amino}-ethyl)-guanidine
- N,N,N',N'-Tetramethyl-N"-(2-{2-[2-(N',N',N",N"-tetramethyl-guanidino)-ethoxy]-ethoxy}-ethyl)-guanidine
- 2-{3-[(1,3-Dimethyl-imidazolidin-2-ylideneamino)-methyl]-cyclohexylmethylimino}-1,3-dimethyl-imidazolidine
- 2-{2-[2-(1,3-Dimethyl-imidazolidin-2-ylideneamino)-ethylsulfanyl]-ethylimino}-1,3-dimethyl-imidazolidine
- N-(1,3-Dimethyl-1,3-dihydro-imidazol-2-ylidene)-N'-[2-(1,3-dimethyl-1,3-dihydro-imidazol-2-ylideneamino)-ethyl]-N'-methyl-ethane-1,2-diamine
- 2-{2-[2-(1,3-Dimethyl-1,3-dihydro-imidazol-2-ylideneamino)-ethyldisulfanyl]-ethylimino}-1,3-dimethyl-2,3-dihydro-1H-imidazole
- N1,N5-Bis-(1,3-dimethyl-tetrahydro-pyrimidin-2-ylidene)-2-methyl-pentane-1,5-diamine
- 2-{3-[(1,3-Dimethyl-tetrahydro-pyrimidin-2-ylideneamino)-methyl]-benzylimino}-1,3-dimethyl-hexahydro-pyrimidine

Les nouveaux composés de formule générale (II) sont préparés suivant les schémas de synthèse décrits pour les composés de formule générale (I).

L'invention concerne également l'utilisation en cosmétique, notamment en cosmétique capillaire et surtout en défrisage, des composés de formule (II) ainsi qu'un procédé cosmétique, notamment cosmétique capillaire et surtout défrisage, mettant en oeuvre au moins un composé de formule (II).

Les composés de formule (II) peuvent être utilisés pour toute matière kératinique, notamment pour les cheveux et la peau, en tant que produit de soin, de protection de maquillage, de maintien. Par exemple, ils peuvent être utilisés dans des rouges à lèvres, des crèmes pour le corps ou les mains, des ombres à paupières, des mascaras, des eye liners ou des fards à joues.

Dans les compositions selon l'invention destinées à un processus de défrisage, décrêpage ou lissage des cheveux, la multiguanidine n'appartenant pas à la famille des hydroxydes est avantageusement présente à une concentration molaire comprise entre 0,1M et 2M, ce qui correspond à des concentrations comprises entre 1,4 % et 80% en poids par rapport au poids total de la composition, et plus avantageusement, à une concentration comprise entre 0,2M et 1M, ce qui correspond à des concentrations comprises entre 2,8 % et 40 % en poids par rapport au poids total de la composition.

Dans des compositions cosmétiques, notamment des compositions de défrisage, les concentrations molaires et pondérales des composés de formule générale (II) sont les mêmes que pour les composés de formule générale (I)

Le pH des compositions selon l'invention est, de préférence, compris entre 9,6 et 14, et plus particulièrement compris entre 11 et 13.

Avantageusement, dans les compositions de l'invention, la multiguanidine n'appartenant pas à la famille des hydroxydes constitue le seul actif de défrisage.

Les compositions selon l'invention peuvent également contenir des agents réducteurs connus, tels que par exemple l'acide thioglycolique ou l'acide thiolactique et leurs dérivés esters et amides, notamment le monothioglycolate de glycérol, la cystéamine et ses dérivés acylés en C1-C4 tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides décrits dans la demande de brevet EP-A-354 835, les N-mono ou N,N-dialkylmercapto-4-butyramides décrits dans le demande de brevet EP-A-368 763, les aminomercaptoalkylamides, décrits dans le demande de brevet EP-A-432 000, les dérivés des acides N-(mercaptoalkyl)succinamiques et des N-(mercaptoalkyl)succinimides décrits dans le demande de brevet EP-A-465 342, les alkylamino mercaptoalkylamides décrits dans la demande de brevet EP-A-514 282, le mélange azéotrope de thioglycolate de 2-hydroxypropyle et de thioglycolate de (2-hydroxy-1-méthyl)éthyle décrits dans la demande de brevet FR-A-2 679 448, les mercaptoalkylaminoamides décrits dans la demande de brevet FR-A-2 692 481, les N-mercaptoalkylalcanediamides décrits dans le demande de brevet EP-A-653 202, ainsi que les dérivés d'acide formamidine sulfinique décrits dans la demande PCT/US01/43124, déposée par la Demanderesse.

Lorsque les compositions selon l'invention contiennent au moins un agent réducteur, celui-ci est avantageusement présent à une concentration maximale de 20% en poids, et de préférence, comprise entre 0,1 et 10% en poids par rapport au poids total de la composition.

Les compositions selon l'invention peuvent également contenir des hydroxydes connus choisis de préférence parmi les hydroxydes de métaux alcalins ou alcalino-terreux ou de métaux de transition ou organiques tels que l'hydroxyde de sodium, l'hydroxyde de potassium, l'hydroxyde de lithium, l'hydroxyde de rubidium, l'hydroxyde de césium, l'hydroxyde de francium, l'hydroxyde de béryllium, l'hydroxyde de magnésium, l'hydroxyde de calcium, l'hydroxyde de strontium, l'hydroxyde de barium, l'hydroxyde de molybdenium, l'hydroxyde de manganèse, l'hydroxyde de zinc, l'hydroxyde de cobalt, l'hydroxyde de cadmium, l'hydroxyde de cérium, l'hydroxyde de lanthanium, l'hydroxyde d'actinium, l'hydroxyde de thorium, l'hydroxyde d'aluminium, l'hydroxyde de guanidinium et les hydroxydes d'ammoniums quaternaires.

Lorsque les compositions de l'invention contiennent au moins un hydroxyde, celui-ci est avantageusement présent à une concentration allant de 0,01 % à 3,5 % en poids, et de préférence, comprise entre 0,05 et 1,5 % en poids par rapport au poids total de la composition.

Selon un mode de réalisation particulièrement avantageux des compositions selon l'invention, ces dernières contiennent 0 % de base appartenant à la famille des hydroxydes, choisie notamment parmi les hydroxydes de métaux alcalins ou alcalino-terreux ou de métaux de transition ou organiques.

Avantageusement, les compositions de l'invention comprennent entre 0 et 50 % d'eau, de préférence entre 0 et 30%, et plus préférentiellement encore entre 0 et 20%.

Selon un mode de réalisation préféré, les compositions basiques contiennent également un agent tensioactif de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque les compositions basiques contiennent au moins un agent tensioactif, celui-ci est généralement présent à une concentration maximale de 30 % en poids, et de préférence, comprise entre 0,5 et 10 % en poids par rapport au poids total de la composition.

Dans le but d'améliorer les propriétés cosmétiques des cheveux ou encore d'en atténuer ou d'éviter leur dégradation, la composition basique peut également contenir un agent traitant de nature cationique, anionique, non-ionique ou amphotère.

Parmi les agents traitants particulièrement préférés, on peut notamment citer ceux décrits dans les brevets français n° 2.598.613 et n° 2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxane- polyoxyalkyle du type Diméthicone Copolyol, un polydiméthylsiloxane à groupements terminaux stéaroxy (stéaroxydiméthicone), un copolymère polydiméthylsiloxane- dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

Les compositions basiques selon l'invention peuvent également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n°83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de féthylèneglycol et le monoéthyléther du diéthylèneglycol, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants ou complexants, des agents opacifiants, des filtres solaires ainsi que des parfums et des conservateurs.

Avantageusement, dans les compositions selon l'invention, la multiguanidine n'appartenant pas à la famille des hydroxydes défrise des fibres kératiniques sans être mise en contact, au préalable, avec un solvant organique.

Les compositions selon l'invention se présentent essentiellement sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui "collent" les cheveux et les maintiennent dans la position lisse pendant le temps de pose.

Les compositions selon l'invention peuvent contenir, en outre, au moins un adjuvant choisi parmi les silicones sous forme soluble, dispersée, micro-dispersée, les agents tensio-actifs non-ioniques, anioniques, cationiques et amphotères, les céramides, les glycocéramides et pseudo-céramides, les vitamines et pro-vitamines dont le panthénol, les huiles végétales, animales, minérales et synthétiques, les cires autres que les céramides, les glycocéramides et pseudo-céramides, les filtres solaires hydrosolubles et liposolubles, siliconés ou non siliconés, les agents nacrants et opacifiants, les agents séquestrants, les agents plastifiants, les agents solubilisants, les agents acidifiants, les agents épaississants minéraux et organiques, les agents anti-oxydants, les hydroxyacides, les agents de pénétration, les parfums et les agents conservateurs. Parmi les agents solubilisants, on peut par exemple citer les alcools inférieurs tels que par exemple l'éthanol, le propanol ou l'isopropanol.

L'invention concerne également un kit comprenant au moins deux compartiments, l'un des compartiments (i) comprenant, dans un milieu cosmétiquement acceptable, au moins une multiguanidine n'appartenant pas à la famille des hydroxydes apte à réagir sur les cystines des fibres kératiniques, par une réaction de bêta-élimination produisant de la déhydro-alanine et conduisant à la formation de lanthionine, pour défriser les fibres kératiniques en moins de 60 minutes.

De préférence, le kit selon l'invention, comprend, en outre, une composition supplémentaire (ii) de soin, de conditionnement, de maquillage, de démaquillage, de protection, de nettoyage ou de lavage des fibres kératiniques.

Les compositions des kits selon l'invention sont conditionnées dans des compartiments ou récipients ou dispositifs distincts, accompagnés, éventuellement, de moyens d'application appropriés, identiques ou différents, tels que les pinceaux, les brosses, les éponges.

Un autre objet de l'invention concerne un procédé défrisage des matières kératiniques mettant en oeuvre une composition cosmétique comprenant, dans un milieu cosmétiquement acceptable, au moins une multiguanidine n'appartenant pas à la famille des hydroxydes, le milieu cosmétiquement acceptable et la multiguanidine étant choisis de façon telle que la multiguanidine n'appartenant pas à la famille des hydroxydes réagisse sur les cystines des fibres kératiniques, par une réaction de bêta-élimination produisant de la déhydro-alanine et conduisant à la formation de lanthionine, pour défriser les fibres kératiniques en moins de 60 minutes.

Avantageusement, dans le procédé selon l'invention, la durée du défrisage est inférieure à 40 minutes, et plus avantageusement inférieure à 30 minutes

Dans le procédé de défrisage ou de décrêpage ou de lissage des cheveux selon l'invention, on applique sur les cheveux la composition basique selon l'invention, puis l'on soumet les cheveux à une déformation mécanique permettant de leur conférer une nouvelle forme, par une opération de lissage des cheveux avec un peigne à larges dents, avec le dos d'un peigne ou à la main. Après un temps de pose de 5 à 60 minutes, en particulier, de 5 à 40 minutes, on procède alors à un nouveau lissage, puis on rince abondamment les cheveux.

Selon la présente invention, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de lissage de la chevelure, un fer chauffant à une température comprise entre 60 et 220°C et de préférence entre 120 et 200°C.

Encore un autre objet de l'invention concerne l'utilisation d'une multiguanidine n'appartenant pas à la famille des hydroxydes comme agent actif de défrisage des fibres kératiniques.

L'invention concerne aussi un agent actif de défrisage des fibres kératiniques, au moyen d'une réaction de béta-élimination produisant de la déhydro-alanine et conduisant à la formation de lanthionine, comprenant au moins une multiguanidine n'appartenant pas à la famille des hydroxydes.

L'invention pourra être mieux comprise à l'aide des exemples non limitatifs qui suivent et qui constituent des modes de réalisation préférentiels des compositions selon l'invention.

### EXEMPLE 1:

On réalise une composition de défrisage simplifiée contenant le N",N""'-1,3-propanediylbis[N,N,N',N'-tetramethyl-guanidine](RN :289474-28-8) à une concentration de 0,5M dans l'eau, en tant qu'actif de défrisage. On applique cette composition sur des cheveux africains naturellement frisés pendant 20 minutes à une température de 30°C. Les cheveux sont défrisés efficacement, faciles à peigner et à coiffer et doux au toucher.

### EXEMPLE 2:

On réalise une composition de défrisage simplifiée contenant le N",N""',N""""-(nitrilotri-2,1-ethanediyl)tris[N,N,N',N'-tetramethyl-guanidine] (RN: 368866-05-1) à une concentration de 0,25M dans l'eau, en tant qu'actif de défrisage. On applique cette composition sur des cheveux africains naturellement frisés pendant 25 minutes à une température de 30°C. Les cheveux sont défrisés efficacement, faciles à peigner et à coiffer et doux au toucher.

## Revendications

1. Composition cosmétique comprenant, dans un milieu cosmétiquement acceptable, au moins une multiguanidine n'appartenant pas à la famille des hydroxydes, le milieu cosmétiquement acceptable et la multiguanidine étant choisis de façon telle que la multiguanidine n'appartenant pas à la famille des hydroxydes réagisse sur les cystines des fibres kératiniques, par une réaction de bêta-élimination produisant de la déhydro-alanine et conduisant à la formation de lanthionine, pour défriser les fibres kératiniques en moins de 60 minutes.

2. Composition selon la revendication 1, **caractérisée par** le fait la durée du défrisage est inférieure à 40 minutes, et plus avantageusement inférieure à 30 minutes.

3. Composition selon l'une quelconque des revendications précédentes, **caractérisée par le fait que** la multiguanidine n'appartenant pas à la famille des hydroxydes est avantageusement présente à une concentration molaire comprise entre 0,1M et 2M, et plus avantageusement, à une concentration comprise entre 0,2M et 1M.

4. Composition selon l'une quelconque des revendications précédentes, **caractérisée par le fait que** le pH est compris entre 9,6 et 14, et plus particulièrement compris entre 11 et 13.

5. Composition selon l'une quelconque des revendications précédentes, **caractérisée par le fait qu'**elle contient 0 % de base appartenant à la famille des hydroxydes.

6. Composition selon l'une quelconque des revendications précédentes, **caractérisée par le fait qu'**elle comprend entre 0 et 50 % d'eau, de préférence entre 0 et 30%, et plus préférentiellement encore entre 0 et 20 %.

7. Composition selon l'une quelconque des revendications précédentes, **caractérisée par le fait que** la multiguanidine est choisie dans le groupe des composés répondant à la formule générale(I) suivante : 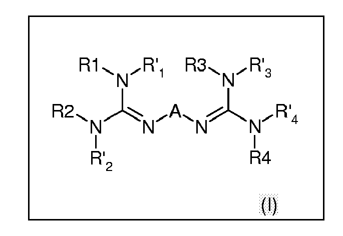 dans laquelle :
• R1,R'1,R2,R'2,R3,R'3,R4,R'4, identiques ou différents représentent un radical choisi parmi :
- un atome d'hydrogène
- un groupement alkyle en C1 à C6, saturé ou insaturé, linéaire tel que par exemple méthyle ou éthyle, ou ramifié tel que par exemple isopropyle ou tertbutyle, ou encore cyclique tel que par exemple cyclopentyle ou cyclohexyle, ledit groupement alkyle étant éventuellement substitué par le radical suivant : 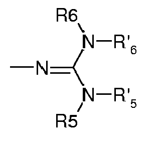 dans lequel R5,R'5,R6 et R'6 ont les mêmes significations que les radicaux R1 à R'4 désignés ci-dessus,
• R1et R2 et/ou R3 et R4 peuvent également former ensemble un radical divalent (CH2)2 ou (CH2)3 ou CH=CH.
• A représente un radical hydrocarboné divalent en C2 à C12, linéaire ou cyclique, saturé ou insaturé , pouvant être éventuellement interrompu par un ou plusieurs groupes tels que imino,carboxamido, sulfoxyde, sulfone, et/ou par :
- un ou plusieurs hétéoatomes choisis parmi le soufre, l'oxygène, l'azote et le silicium et éventuelllement substitué par :
- un radical alkyle en C1 à C4 linéaire ou ramifié éventuellement interrompu par un ou plusieurs hétéroatomes tels que définis ci-dessus et éventuelllement substitué par le radical suivant : 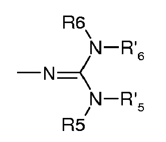 dans lequel R5,R'5,R6 et R'6 ont les mêmes significations que les radicaux R1 à R'4 désignés ci-dessus.

8. Composition selon l'une quelconque des revendications précédentes, **caractérisée par le fait que** les composés de formule (I) sont choisis parmi:
- N,N'-bis(1,3-dimethyl-2-imidazolidinylidene)-1,2-benzenediamine (RN: 774610-65-0)
- N-[bis(dimethylamino)methylene]-3-(dimethylamino)-5-imino-2-methyl-2,4,6,19-Tetraazaeicosan-20-imidamide (RN : 791543-84-5)
- N,N'-bis(tetrahydro-1,3-dimethyl-2(1H)-pyrimidinylidene)-1,3-propanediamine (RN :752232-69-2)
- 1,1',1",1"'-[1propanediylbis(nitrilomethanetetrayl)]tetrakis-piperidine (RN : 752232-68-1)
- Poly(oxy-1,2-ethanediyl), α-[2-[(1,3-dimethyl-2-imidazolidinylidene)amino]ethyl]-ω-[2-[(1,3-dimethyl-2-imidazolidinylidene)amino]ethoxy] (RN : 742679-23-8)
- N",N""'-1,2-ethanediylbis[N,N,N',N'-tetrakis(1-methylethyl)-guanidine (RN : 676488-06-5)
- N,N'-bis(1,3-dihydro-1,3-dimethyl-2H-imidazol-2-ylidene)-1,8-naphtalenediamine (RN : 634192-99-7)
- N,N'-bis(1,3-dimethyl-2-imidazolidinylidene)- 1,8-Naphthalenediamine (RN : 501931-38-0)
- N'',N'''''-1,8-biphenylenediylbis[N,N,N',N'-tetramethyl-guanidine] (RN : 495408-66-7)
- N",N""'-benzo[c]phenanthrene-1,12-diylbis[N,N,N',N'-tetramethyl-guanidine] (RN : 495408-58-7)
- N",N'''''-4,5-phenanthrenediylbis[N,N,N',N'-tetramethyl-guanidine] (RN : 495408-50-9)
- N",N""'-(9,10-dihydro-4,5-phenanthrenediyl)bis[N,N,N',N'-tetramethylguanidine] (RN : 495408-44-1)
- N'',N'''''-1,2-phenylenebis[N,N,N',N'-tetramethyl-guanidine] (RN : 495408-35-0)
- N'',N'''''-1,8-naphthalenediylbis[N,N,N',N'-tetramethyl-guanidine] hydrochloride (RN : 443892-20-4)
- N",N""'-9H-fluorene-4,5-diylbis[N,N,N',N'-tetramethyl-guanidine] (RN : 443892-12-4)
- N'-(tetrahydro-1,3-dimethyl-2(1H)-pyrimidinylidene)-N,N-bis[2-[(tetrahydro-1,3-dimethyl-2(1H)-pyrimidinylidene)amino]ethyl]-1,2-ethanediamine (RN : 395640-62-7)
- N'',N''''',N''''''''-(nitrilotri-2,1-ethanediyl)tris[N,N,N',N'-tetramethyl-guanidine] (RN : 368866-05-1)
- N'',N'''''-[2-[[[bis(dimethylamino)methylene]amino]methyl]-2-methyl-1,3-propanediyl]bis[N,N,N',N'-tetramethyl-guanidine] (RN : 289474-30-2)
- N'',N'''''-(2,2-dimethyl-1,3-propanediyl)bis[N,N,N',N'-tetramethyl-guanidine] (RN : 289474-29-9)
- N",N""'-1,3-propanediylbis[N,N,N',N'-tetramethyl-guanidine](RN :289474-28-8)
- N",N""'-1,2-ethanediylbis[N,N,N',N'-tetramethyl guanidine] (RN : 289474-25-5)
- N,N'-bis(1,3-dihydro-1,3-dimethyl-2H-imidazol-2-ylidene)-1,2-Ethanediamine (RN : 216873-26-6)
- N,N"'-1,2-ethanediylbis[N',N',N",N"-diethyl-guanidine] (RN : 211869-99-7)
- N",N""'-[(methylimino)di-3,1-propanediyl]bis[N,N,N',N'-tetramethylguanidine] (RN : 196405-86-4)
- N",N'''''-[oxybis(2,1-ethanediyloxy-3,1-propanediyl)]bis[N,N,N',N'-tetramethylguanidine] (RN : 190442-53-6)
- N",N'''''-[[1,1,3,3-tetramethyl-1,3-disiloxanediyl]di-3,1-propanediyl]bis[N,N,N',N'-tetramethylguanidine] (RN : 175989-14-7)
- N",N'""-[[1,3-dimethyl-1,3-bis[(trimethylsilyl)oxy]-1,3-disiloxanediyl]di-3,1-propanediyl]bis[N,N,N',N'-tetramethyl-guanidine] (RN : 172283-48-6)
- N,N"-1,6-hexanediylbis[N'-[bis(dimethylamino)methylene urea (RN : 157362-45-3)
- [bis(dimethylamino)methylene] [3[[[[[bis(dimethylamino)methyl ene]amino]carbonyl]amino]methyl]-3,5,5-trimethylcyclohexyl]-urea (RN: 157362-44-2)
- N",N""'-[1,1'-biphenyl]-2,2'-diylbis[N,N,N',N'-tetramethyl-guanidine] (RN: 144576-63-6)
- 4,4'-sulfonylbis[N-(1,3-dimethyl-2-imidazolidinylidene)-benzenamine (RN : 129346-76-5)
- N,N'-bis(1,3-dimethyl-2-imidazolidinylidene),2,8-dibenzothiophenediamine-5,5-dioxide (RN : 128169-35-7)
- N,N'-bis(1,3-dimethyl-2-imidazolidinylidene)-4,6-Dibenzothiophenediamine -5,5-dioxide (RN : 127330-56-7)
- N,N'-bis(1,3-dimethyl-2-imidazolidinylidene)- 1,2-Ethanediamine (RN : 126620-51-7)
- N'',N'''''-1,3-phenylenebis[N,N,N',N'-tetramethyl-guanidine] (RN : 121648-84-8)
- N,N'-bis[bis(dimethylamino)methylene]-butanediamine (RN : 114491-72-4)
- N'',N'''''-1,4-phenylenebis[N,N,N',N'-tetramethyl -guanidine] (RN: 113551-45-4)
- N'',N'''''-[[1,3-dimethyl-1-[(pentamethyldisiloxanyl)oxy]-3-[(trimethylsilyl)oxy]- 1,3-disiloxanediyl]di-3,1-propanediyl]bis[N,N,N',N'-tetramethylguanidine]
(RN : 109956-31-2)
- N,N"-bis[(dimethylamino)(dipentylamino)methylene]-1,4-Piperazinedicarboximidamide (RN : 97983-93-2)
- ,N,N"-bis[(dimethylamino)(hexylpropylamino)methylene]-1,4-Piperazinedicarboximidamide (RN : 97983-92-1)
- N,N"-bis[(hexylmethylamino)(methylpropylamino)methylene]-1,4-Piperazinedicarboximidamide (RN : 97983-91-0)
- N,N"-bis[(butylmethylamino)(hexylmethylamino)methylene]-1,4-Piperazinedicarboximidamide (RN : 97983-90-9)
- N,N"-bis[(dimethylamino)(heptylmethylamino)methylene]-1,4-Piperazinedicarboximidamide (RN : 97963-91-2)
- N'',N'''''-[(1,1,3,3-tetramethoxy-1,3-disiloxanediyl)di-3,1-propanediyl]bis[N,N,N',N'-tetramethyl-guanidine] (RN : 69755-28-8)
- N'',N'''''-1,6-hexanediylbis[N,N,N',N'-tetramethyl-guanidine] (RN : 64933-93-3)
- N'',N'''''-(methylenedi-4,1-phenylene)bis [N,N,N',N'-tetramethyl-guanidine] (RN : 57414-23-0)
- N,N"-(methylphenylene)bis[N'-[bis(dimethylamino)methylene]-urea (RN : 39529-23-2)
- 2,2'-(sulfonyldiethylene)bis[1,1,3,3-tetramethyl guanidine] (RN : 13998-89-5)

9. Composition selon l'une quelconque des revendications 7 ou 8, **caractérisée par le fait que** les composés de formule générale (I) sont choisis parmi:
- N-[bis(dimethylamino)methylene]-3-(dimethylamino)-5-imino-2-methyl-2,4,6,19-Tetraazaeicosan-20-imidamide (RN : 791543-84-5)
- N,N'-bis(tetrahydro-1,3-dimethyl-2(1H)-pyrimidinylidene)-1,3-propanediamine(RN :752232-69-2)
- Poly(oxy-1,2-ethanediyl), α-[2-[(1,3-dimethyl-2-imidazolidinylidene)amino]ethyl]-ω-[2-[(1,3-dimethyl-2-imidazolidinylidene)amino]ethoxy] (RN : 742679-23-8)
- N'',N'''''-1,2-ethanediylbis[N,N,N',N'-tetrakis(1-methylethyl)-guanidine (RN : 676488-06-5)
- N,N'-bis(1,3-dihydro-1,3-dimethyl-2H-imidazol-2-ylidene)-1,8-naphtalenediamine (RN : 634192-99-7)
- N,N'-bis(1,3-dimethyl-2-imidazolidinylidene)- 1,8-Naphthalenediamine (RN : 501931-38-0)
- N",N""'-1,2-phenylenebis[N,N,N',N'-tetramethyl-guanidine] (RN : 495408-35-0)
- N",N""'-1,8-naphthalenediylbis[N,N,N',N'-tetramethyl-guanidine] hydrochloride (RN : 443892-20-4)
- N",N""',N""""-(nitrilotri-2,1-ethanediyl)tris[N,N,N',N'-tetramethyl- guanidine] (RN : 368866-05-1)
- N",N""'-[2-[[[bis(dimethylamino)methylene]amino]methyl]-2-methyl-1,3-propanediyl]bis[N,N,N',N'-tetramethyl-guanidine] (RN : 289474-30-2)
- N",N""'-(2,2-dimethyl-1,3-propanediyl)bis[N,N,N',N'-tetramethyl-guanidine] (RN : 289474-29-9)
- N'',N'''''-1,3-propanediylbis[N,N,N',N'-tetramethyl-guanidine](RN :289474-28-8)
- N,N'-bis(1,3-dihydro-1,3-dimethyl-2H-imidazol-2-ylidene)-1,2- Ethanediamine (RN : 216873-26-6)
- N,N'''-1,2-ethanediylbis[N',N',N'',N''-diethyl-guanidine] (RN : 211869-99-7)
- N",N''''''-[(methylimino)di-3,1-propanediyl]bis[N,N,N',N'-tetramethylguanidine] (RN : 196405-86-4)
- N'',N'''''-[oxybis(2,1-ethanediyloxy-3,1-propanediyl)]bis[N,N,N',N'-tetramethylguanidine] (RN : 190442-53-6)
- N'',N'''''-[[1,1,3,3-tetramethyl-1,3-disiloxanediyl]di-3,1-propanediyl]bis[N,N,N',N'-tetramethylguanidine (RN : 175989-14-7)
- N,N"-1,6-hexanediylbis[N'-[bis(dimethylamino)methylene urea (RN : 157362-45-3)
- N",N""'-[1,1'-biphenyl]-2,2'-diylbis[N,N,N',N'-tetramethyl-guanidine] (RN: 144576-63-6)
- N,N'-bis(1,3-dimethyl-2-imidazolidinylidene)- 1,2-Ethanediamine (RN : 126620-51-7)
- N",N""'-1,4-phenylenebis[N,N,N',N'-tetramethyl ―guanidine] (RN: 113551-45-4)
- N",N""'-[(1,1,3,3-tetramethoxy-1,3-disiloxanediyl)di-3,1-propanediyl]bis[N,N,N',N'-tetramethyl-guanidine] (RN : 69755-28-8)
- N",N""'-1,6-hexanediylbis[N,N,N',N'-tetramethyl-guanidine] (RN : 64933-93-3)
- 2,2'-(sulfonyldiethylene)bis[1,1,3,3-tetramethyl guanidine] (RN : 13998-89-5)

10. Composés de formule générale (II) : 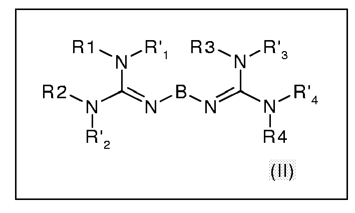 Dans laquelle :
• R1,R'1,R2,R'2,R3,R'3,R4,R'4, identiques ou différents représentent un groupement choisi parmi :
- un atome d'hydrogène
- un radical méthyle, éthyle, propyle ou isopropyle
R1et R2 et/ou R3 et R4 peuvent également former ensemble un radical divalent (CH2)2 ou (CH2)3 ou CH=CH.
B représente un radical choisi parmi : 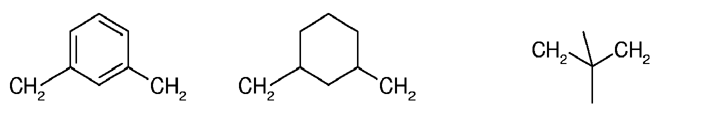 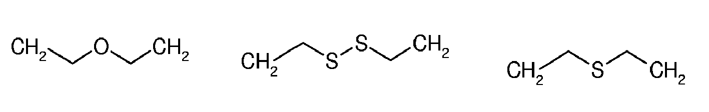 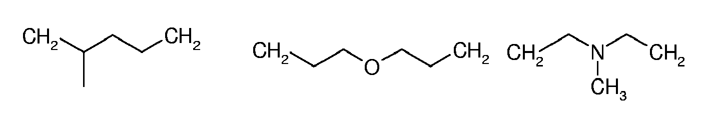 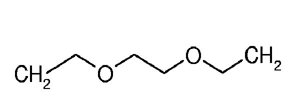
• Lorsque R'1 =R'2= R'3= R'4 = CH3 et lorsque R1/R2 et R3/R4 forment respectivement et simultanément le radical divalent (CH2)2 ;
B représente également un radical (CH2)2 ou (CH2)5 ou (CH2)6 ou un radical : 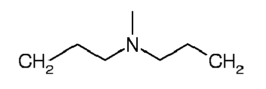
• Lorsque R'1 =R'2= R'3= R'4 = CH3 et lorsque R1/R2 et R3/R4 forment respectivement et simultanément le radical divalent (CH2)3;
B représente (CH2)2 ou (CH2)3 ou (CH2)4 ou (CH2)5 ou (CH2)6 ou un radical : 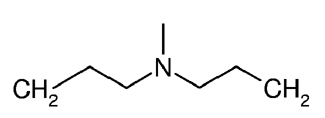
• Lorsque R'1 =R'2= R'3= R'4 = CH3 et lorsque R1/R2 et R3/R4 forment respectivement et simultanément le radical divalent CH=CH :
B représente (CH2)3 ou (CH2)4 ou (CH2)5 ou (CH2)6 ou un radical : 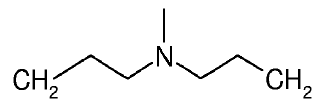
• Lorsque R1,R'1,R2,R'2,R3,R'3,R4,R'4 représentent un radical ethyle ou isopropyle, B représente un radical (CH2)3 ou (CH2)4 ou (CH2)5 ou (CH2)6 ou un radical : 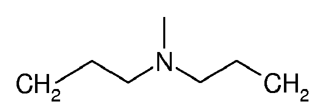
• Lorsque R1,R'1,R2,R'2,R3,R'3,R4,R'4 représentent un radical méthyle, B représente un radical (CH2)4 ou (CH2)5.

11. Composés de formule générale (II) selon la revendication 10 choisis parmi:
• N,N,N',N'-Tetraisopropyl-N"-[3-(N',N',N",N"-tetraisopropyl-guanidino)-propyl]-guanidine
• N,N,N',N'-Tetraisopropyl-N"-[4-(N',N',N",N"-tetraisopropyl-guanidino)-butyl]-guanidine
• N,N,N',N'-Tetraisopropyl-N"-[5-(N',N',N",N"-tetraisopropyl-guanidino)-pentyl]-guanidine
• N,N,N',N'-Tetraisopropyl-N"-[6-(N',N',N",N"-tetraisopropyl-guanidino)-hexyl]-guanidine
• N,N,N',N'-Tetraisopropyl-N''-(3-{methyl-[3-(N',N',N'',N''-tetraisopropylguanidino)-propyl]-amino}-propyl)-guanidine
• N,N,N',N'-Tetraethyl-N"-[3-(N',N',N",N"-tetraethyl-guanidino)-propyl]-guanidine
• N,N,N',N'-Tetraethyl-N"-[4-(N',N',N",N"-tetraethyl-guanidino)-butyl]-guanidine
• N,N,N',N'-Tetraethyl-N"-[5-(N',N',N",N"-tetraethyl-guanidino)-pentyl]-guanidine
• N,N,N',N'-Tetraethyl-N"-[6-(N',N',N",N"-tetraethyl-guanidino)-hexyl]-guanidine
• N,N,N',N'-Tetraethyl-N''-(3-{methyl-[3-(N',N',N",N"-tetraisopropyl-guanidino)-propyl]-amino}-propyl)-guanidine
• N,N'-Bis-(1,3-dimethyl-imidazolidin-2-ylidene)-butane-1,4-diamine
• N,N'-Bis-(1,3-dimethyl-imidazolidin-2-ylidene)-pentane-1,5-diamine
• N,N'-Bis-(1,3-dimethyl-imidazolidin-2-ylidene)-hexane-1,6-diamine
• N-(1,3-Dimethyl-imidazolidin-2-ylidene)-N'-[3-(1,3-dimethyl-imidazolidin-2-ylideneamino)-propyl]-N'-methyl-propane-1,3-diamine
• N,N'-Bis-(1,3-dimethyl-1,3-dihydro-imidazol-2-ylidene)-propane-1,3-diamine
• N,N'-Bis-(1,3-dimethyl-1,3-dihydro-imidazol-2-ylidene)-butane-1,4-diamine
• N,N'-Bis-(1,3-dimethyl-1,3-dihydro-imidazol-2-ylidene)-pentane-1,5-diamine
• N,N'-Bis-(1,3-dimethyl-1,3-dihydro-imidazol-2-ylidene)-hexane-1,6-diamine
• N-(1,3-Dimethyl-1,3-dihydro-imidazol-2-ylidene)-N'-[3-(1,3-dimethyl-1,3-dihydro-imidazol-2-ylideneamino)-propyl]-N'-methyl-propane-1,3-diamine
• N,N'-Bis-(1,3-dimethyl-tetrahydro-pyrimidin-2-ylidene)-ethane-1,2-diamine
• N,N'-Bis-(1,3-dimethyl-tetrahydro-pyrimidin-2-ylidene)-propane-1,3-diamine
• N,N'-Bis-(1,3-dimethyl-tetrahydro-pyrimidin-2-ylidene)-butane-1,4-diamine
• N,N'-Bis-(1,3-dimethyl-tetrahydro-pyrimidin-2-ylidene)-pentane-1,5-diamine
• N,N'-Bis-(1,3-dimethyl-tetrahydro-pyrimidin-2-ylidene)-hexane-1,6-diamine
• N-(1,3-Dimethyl-tetrahydro-pyrimidin-2-ylidene)-N'-[3-(1,3-dimethyltetrahydro-pyrimidin-2-ylideneamino)-propyl]-N'-methyl-propane-1,3-diamine
• N,N,N',N'-Tetramethyl-N"-[4-(N',N',N",N"-tetramethyl-guanidino)-butyl]-guanidine
• N,N,N',N'-Tetramethyl-N"-[5-(N',N',N",N"-tetramethyl-guanidino)-pentyl]-guanidine
• N,N,N',N'-Tetramethyl-N''-[3-(N',N',N'',N''-tetramethyl-guanidinomethyl)-cyclohexylmethyl]-guanidine
• N,N,N',N'-Tetraethyl-N''-[3-(N',N',N'',N''-tetraethyl-guanidinomethyl)-cyclohexylmethyl]-guanidine
• N,N,N',N'-Tetrapropyl-N"-[3-(N',N',N",N"-tetrapropyl-guanidinomethyl)-cyclohexylmethyl]-guanidine
• N,N,N',N'-Tetraisopropyll-N"-[3-(N',N',N",N"-tetraisopropyl-guanidinomethyl)-cyclohexylmethyl]-guanidine
• N,N,N',N'-Tetraethyl-N"-[3-(N',N',N",N"-tetraethyl-guanidinomethyl)-benzyl]-guanidine
• N,N,N',N'-Tetrapropyll-N"-[3-(N',N',N",N"-tetrapropyl-guanidinomethyl)-benzyl]-guanidine
• N,N,N',N'-Tetraisopropyl-N"-[3-(N',N',N",N"-tetraisopropyl-guanidinomethyl)-benzyl]-guanidine
• N"-[2,2-Dimethyl-3-(N',N',N",N"-tetraethyl-guanidino)-propyl]-N,N,N',N'-tetraethyl-guanidine
• N"-[2,2-Dimethyl-3-(N',N',N",N"-tetrapropyl-guanidino)-propyl]-N,N,N',N'-tetrapropyl-guanidine
• N"-[2,2-Dimethyl-3-(N',N',N",N"-tetraisopropyl-guanidino)-propyl]-N,N,N',N'-tetraisopropyl-guanidine
• N,N,N',N'-Tetramethyl-N''-{2-[2-(N',N',N'',N''-tetramethyl-guanidino)-ethoxy]-ethyl}-guanidine
• N,N,N',N'-Tetraethyl-N''-{2-[2-(N',N',N'',N''-tetraethyl-guanidino)-ethoxy]-ethyl}-guanidine
• N,N,N',N'-Tetrapropyl-N''-{2-[2-(N',N',N'',N''-tetrapropyl-guanidino)-ethoxy]-ethyl}-guanidine
• N,N,N',N'-Tetraisopropyl-N"-{2-[2-(N',N',N",N"-tetraisopropyl-guanidino)-ethoxy]-ethyl}-guanidine
• N,N,N',N'-Tetramethyl-N"-{2-[2-(N',N',N",N"-tetramethyl-guanidino)-ethylsulfanyl]-ethyl}-guanidine
• N,N,N',N'-Tetraethyl-N"-{2-[2-(N',N',N",N"-tetraethyl-guanidino)-ethylsulfanyl]-ethyl}-guanidine
• N,N,N',N'-Tetrapropyl-N"-{2-[2-(N',N',N",N"-tetrapropyl-guanidino)-ethylsulfanyl]-ethyl}-guanidine
• N,N,N',N'-Tetraisopropyl-N"-{2-[2-(N',N',N",N"-tetraisopropyl-guanidino)-ethylsulfanyl]-ethyl}-guanidine
• N,N,N',N'-Tetramethyl-N''-{2-[2-(N',N',N'',N''-tetramethyl-guanidino)-ethyldisulfanyl]-ethyl}-guanidine
• N,N,N',N'-Tetraethyl-N''-{2-[2-(N',N',N'',N''-tetraethyl-guanidino)-ethyldisulfanyl]-ethyl}-guanidine
• N,N,N',N'-Tetrapropyl-N''-{2-[2-(N',N',N'',N''-tetrapropyl-guanidino)-ethyldisulfanyl]-ethyl}-guanidine
• N,N,N',N'-Tetraisopropyl-N''-{2-[2-(N',N',N'',N''-tetraisopropyl-guanidino)-ethyldisulfanyl]-ethyl}-guanidine
• N,N,N',N'-Tetramethyl-N"-[2-methyl-5-(N',N',N",N"-tetramethyl-guanidino)-pentyl]-guanidine
• N,N,N',N'-Tetraethyl-N"-[2-methyl-5-(N',N',N",N"-tetraethyl-guanidino)-pentyl]-guanidine
• N,N,N',N'-Tetrapropyl-N"-[2-methyl-5-(N',N',N",N"-tetrapropyl-guanidino)-pentyl]-guanidine
• N,N,N',N'-Tetraisopropyl-N"-[2-methyl-5-(N',N',N",N"-tetraisopropylguanidino)-pentyl]-guanidine
• N,N,N',N'-Tetramethyl-N"-{3-[3-(N',N',N",N"-tetramethyl-guanidino)-propoxy]-propyl}-guanidine
• N,N,N',N'-Tetraethyl-N"-{3-[3-(N',N',N",N"-tetraethyl-guanidino)-propoxy]-propyl}-guanidine
• N,N,N',N'-Tetrapropyl-N"-{3-[3-(N',N',N",N"-tetrapropyl-guanidino)-propoxy]-propyl}-guanidine
• N,N,N',N'-Tetraisopropyl-N"-{3-[3-(N',N',N",N"-tetraisopropyl-guanidino)-propoxy]-propyl}-guanidine
• N,N,N',N'-Tetramethyl-N"-(2-{methyl-[2-(N',N',N",N"-tetramethyl-guanidino)-ethyl]-amino }-ethyl)-guanidine
• N,N,N',N'-Tetraethyl-N"-(2-{methyl-[2-(N',N',N",N"-tetraethyl-guanidino)-ethyl]-amino }-ethyl)-guanidine
• N,N,N',N'-Tetrapropyl-N"-(2-{methyl-[2-(N',N',N",N"-tetrapropyl-guanidino)-ethyl]-amino}-ethyl)-guanidine
• N,N,N',N'-Tetraisopropyl-N"-(2-{methyl-[2-(N',N',N",N"-tetraisopropylguanidino)-ethyl]-amino}-ethyl)-guanidine
• N,N,N',N'-Tetramethyl-N"-(2-{2-[2-(N',N',N",N"-tetramethyl-guanidino)-ethoxy]-ethoxy }-ethyl)-guanidine
• N,N,N',N'-Tetraethyl-N"-(2-{2-[2-(N',N',N",N"-tetraethyl-guanidino)-ethoxy]-ethoxy}-ethyl)-guanidine
• N,N,N',N'-Tetrapropyl-N"-(2-{2-[2-(N',N',N",N"-tetrapropyl-guanidino)-ethoxy]-ethoxy}-ethyl)-guanidine
• N,N,N',N'-Tetraisopropyl-N"-(2-{2-[2-(N',N',N",N"-tetraisopropyl-guanidino)-ethoxy]-ethoxy}-ethyl)-guanidine
• 2-{3-[(1,3-Dimethyl-imidazolidin-2-ylideneamino)-methyl]-cyclohexylmethylimino}-1,3-dimethyl-imidazolidine
• 2-{3-[(1,3-Dimethyl-imidazolidin-2-ylideneamino)-methyl]-benzylimino}-1,3-dimethyl-imidazolidine
• N,N'-Bis-(1,3-dimethyl-imidazolidin-2-ylidene)-2,2-dimethyl-propane-1,3-diamine
• 2-{2-[2-(1,3-Dimethyl-imidazolidin-2-ylideneamino)-ethylsulfanyl]-ethylimino}-1,3-dimethyl-imidazolidine
• 2-{2-[2-(1,3-Dimethyl-imidazolidin-2-ylideneamino)-ethyldisulfanyl]-ethylimino}-l,3-dimethyl-imidazolidine
• N1,N5-Bis-(1,3-dimethyl-imidazolidin-2-ylidene)-2-methyl-pentane-1,5-diamine
• N-(1,3-Dimethyl-imidazolidin-2-ylidene)-N'-[2-(1,3-dimethyl-imidazolidin-2-ylideneamino)-ethyl]-N'-methyl-ethane-1,2-diamine
• N1,N5-Bis-(1,3-dimethyl-1,3-dihydro-imidazol-2-ylidene)-2-methyl-pentane-1,5-diamine
• N-(1,3-Dimethyl-1,3-dihydro-imidazol-2-ylidene)-N'-[2-(1,3-dimethyl-1,3-dihydro-imidazol-2-ylideneamino)-ethyl]-N'-methyl-ethane-1,2-diamine
• 2-{3-[(1,3-Dimethyl-1,3-dihydro-imidazol-2-ylideneamino)-methyl]-cyclohexylmethylimino }-1,3-dimethyl-2,3-dihydro-1H-imidazole
• N,N'-Bis-(1,3-dimethyl-1,3-dihydro-imidazol-2-ylidene)-2,2-dimethyl-propane-1,3-diamine
• 2-{3-[(1,3-Dimethyl-1,3-dihydro-imidazol-2-ylideneamino)-methyl]-benzylimino }-1,3-dimethyl-2,3-dihydro-1H-imidazole
• 2-{2-[2-(1,3-Dimethyl-1,3-dihydro-imidazol-2-ylideneamino)-ethyldisulfanyl]-ethylimino }-1,3-dimethyl-2,3-dihydro-1H-imidazole
• 2-{2-[2-(1,3-Dimethyl-1,3-dihydro-imidazol-2-ylideneamino)-ethylsulfanyl]-ethylimino}-1,3-dimethyl-2,3-dihydro-1H-imidazole
• N1,N5-Bis-(1,3-dimethyl-tetrahydro-pyrimidin-2-ylidene)-2-methyl-pentane-1,5-diamine
• N-(1,3-Dimethyl-tetrahydro-pyrimidin-2-ylidene)-N'-[2-(1,3-dimethyltetrahydro-pyrimidin-2-ylideneamino)-ethyl]-N'-methyl-ethane-1,2-diamine
• 2-{3-[(1,3-Dimethyl-tetrahydro-pyrimidin-2-ylideneamino)-methyl]-cyclohexylmethylimino }-1,3-dimethyl-hexahydro-pyrimidine
• N,N'-Bis-(1,3-dimethyl-tetrahydro-pyrimidin-2-ylidene)-2,2-dimethyl-propane-1,3-diamine
• 2-{3-[(1,3-Dimethyl-tetrahydro-pyrimidin-2-ylideneamino)-methyl]-benzylimino }-1,3-dimethyl-hexahydro-pyrimidine
• 2-{2-[2-(1,3-Dimethyl-tetrahydro-pyrimidin-2-ylideneamino)-ethyldisulfanyl]-ethylimino}-1,3-dimethyl-hexahydro-pyrimidine
• 2-{2-[2-(1,3-Dimethyl-tetrahydro-pyrimidin-2-ylideneamino)-ethylsulfanyl]-ethylimino }-1,3-dimethyl-hexahydro-pyrimidine

12. Composés de formule générale (II) selon l'une quelconque des revendications 10 ou 11 choisis parmi:
• N,N,N',N'-Tetraisopropyl-N"-[3-(N',N',N",N"-tetraisopropyl-guanidino)-propyl]-guanidine
• N,N,N',N'-Tetraisopropyl-N"-[4-(N',N',N",N"-tetraisopropyl-guanidino)-butyl]-guanidine
• N,N,N',N'-Tetraethyl-N"-[3-(N',N',N",N"-tetraethyl-guanidino)-propyl]-guanidine
• N,N,N',N'-Tetraethyl-N"-[4-(N',N',N",N"-tetraethyl-guanidino)-butyl]-guanidine
• N,N'-Bis-(1,3-dimethyl-imidazolidin-2-ylidene)-butane-1,4-diamine
• N-(1,3-Dimethyl-imidazolidin-2-ylidene)-N'-[3-(1,3-dimethyl-imidazolidin-2-ylideneamino)-propyl]-N'-methyl-propane-1,3-diamine
• N,N'-Bis-(1,3-dimethyl-1,3-dihydro-imidazol-2-ylidene)-propane-1,3-diamine
• N,N'-Bis-(1,3-dimethyl-1,3-dihydro-imidazol-2-ylidene)-butane-1,4-diamine
• N-(1,3-Dimethyl-1,3-dihydro-imidazol-2-ylidene)-N'-[3-(1,3-dimethyl-1,3-dihydro-imidazol-2-ylideneamino)-propyl]-N'-methyl-propane-1,3-diamine
• N,N'-Bis-(1,3-dimethyl-tetrahydro-pyrimidin-2-ylidene)-ethane-1,2-diamine
• N,N'-Bis-(1,3-dimethyl-tetrahydro-pyrimidin-2-ylidene)-propane-1,3-diamine
• N,N,N',N'-Tetramethyl-N''-[4-(N',N',N'',N''-tetramethyl-guanidino)-butyl]-guanidine
• N,N,N',N'-Tetramethyl-N''-[3-(N',N',N'',N''-tetramethyl-guanidinomethyl)-cyclohexylmethyl]-guanidine
• N,N,N',N'-Tetraethyl-N"-[3-(N',N',N",N"-tetraethyl-guanidinomethyl)-benzyl]-guanidine
• N"-[2,2-Dimethyl-3-(N',N',N",N"-tetraethyl-guanidino)-propyl]-N,N,N',N'-tetraethyl-guanidine
• N,N,N',N'-Tetramethyl-N"-{2-[2-(N',N',N",N"-tetramethyl-guanidino)-ethoxy]-ethyl}-guanidine
• N,N,N',N'-Tetramethyl-N"-{2-[2-(N',N',N",N"-tetramethyl-guanidino)-ethylsulfanyl]-ethyl}-guanidine
• N,N,N',N'-Tetramethyl-N"-{2-[2-(N',N',N",N"-tetramethyl-guanidino)-ethyldisulfanyl]-ethyl}-guanidine
• N,N,N',N'-Tetramethyl-N"-[2-methyl-5-(N',N',N",N"-tetramethyl-guanidino)-pentyl]-guanidine
• N,N,N',N'-Tetramethyl-N''-{3-[3-(N',N',N'',N''-tetramethyl-guanidino)-propoxy]-propyl}-guanidine
• N,N,N',N'-Tetramethyl-N''-(2-{methyl-[2-(N',N',N'',N''-tetramethyl-guanidino)-ethyl]-amino }-ethyl)-guanidine
• N,N,N',N'-Tetramethyl-N''-(2-{2-[2-(N',N',N'',N''-tetramethyl-guanidino)-ethoxy]-ethoxy}-ethyl)-guanidine
• 2-{3-[(1,3-Dimethyl-imidazolidin-2-ylideneamino)-methyl]-cyclohexylmethylimino }-1,3-dimethyl-imidazolidine
• 2-{2-[2-(1,3-Dimethyl-imidazolidin-2-ylideneamino)-ethylsulfanyl]-ethylimino }-1,3-dimethyl-imidazolidine
• N-(1,3-Dimethyl-1,3-dihydro-imidazol-2-ylidene)-N'-[2-(1,3-dimethyl-1,3-dihydro-imidazol-2-ylideneamino)-ethyl]-N'-methyl-ethane-1,2-diamine
• 2-{2-[2-(1,3-Dimethyl-1,3-dihydro-imidazol-2-ylideneamino)-ethyldisulfanyl]-ethylimino}-1,3-dimethyl-2,3-dihydro-1H-imidazole
• N1,N5-Bis-(1,3-dimethyl-tetrahydro-pyrimidin-2-ylidene)-2-methyl-pentane-1,5-diamine
• 2-{3-[(1,3-Dimethyl-tetrahydro-pyrimidin-2-ylideneamino)-methyl]-benzylimino}-1,3-dimethyl-hexahydro-pyrimidine

13. Composition selon l'une quelconque des revendications précédentes, **caractérisée par le fait qu'**elle contient, en outre, au moins un adjuvant choisi parmi les silicones sous forme soluble, dispersée, micro-dispersée, les agents tensio-actifs non-ioniques, anioniques, cationiques et amphotères, les céramides, les glycocéramides et pseudo-céramides, les vitamines et pro-vitamines dont le panthénol, les huiles végétales, animales, minérales et synthétiques, les cires autres que les céramides, les glycocéramides et pseudo-céramides, les filtres solaires hydrosolubles et liposolubles, siliconés ou non siliconés, les agents nacrants et opacifiants, les agents séquestrants, les agents plastifiants, les agents solubilisants, les agents acidifiants, les agents épaississants minéraux et organiques, les agents anti-oxydants, les hydroxyacides, les agents de pénétration, les parfums et les agents conservateurs.

14. Kit comprenant au moins deux compartiments, l'un des compartiments (i) comprenant, dans un milieu cosmétiquement acceptable, au moins une multiguanidine n'appartenant pas à la famille des hydroxydes apte à réagir sur les cystines des fibres kératiniques, par une réaction de bêta-élimination produisant de la déhydro-alanine et conduisant à la formation de lanthionine, pour défriser les fibres kératiniques en moins de 60 minutes.

15. Kit selon l'une quelconque des revendications précédentes, **caractérisé par le fait qu'**il comprend, en outre, une composition supplémentaire (ii) de soin, de conditionnement, de maquillage, de démaquillage, de protection, de nettoyage ou de lavage des fibres kératiniques.

16. Procédé de défrisage des matières kératiniques mettant en oeuvre une composition cosmétique comprenant, dans un milieu cosmétiquement acceptable, au moins une multiguanidine n'appartenant pas à la famille des hydroxydes, le milieu cosmétiquement acceptable et la multiguanidine étant choisis de façon telle que la multiguanidine n'appartenant pas à la famille des hydroxydes réagisse sur les cystines des fibres kératiniques, par une réaction de bêta-élimination produisant de la déhydro-alanine et conduisant à la formation de lanthionine, pour défriser les fibres kératiniques en moins de 60 minutes.

17. Procédé de défrisage des matières kératiniques selon la revendication 16, **caractérisé par le fait qu'**après application de la composition, on soumet la chevelure à un traitement thermique par chauffage à une température comprise entre 30 et 60°C.

18. Procédé de défrisage des matières kératiniques selon la revendication 16 ou 17, **caractérisé par le fait qu'**on utilise, à la fois comme moyen de chauffage et de lissage de la chevelure, un fer chauffant à une température comprise entre 60 et 220°C et de préférence entre 120 et 200°C.

19. Procédé de défrisage des matières kératiniques selon la revendication 16 à 18, **caractérisé par le fait que** la durée du défrisage est inférieure à 40 minutes, et plus avantageusement inférieure à 30 minutes.

20. Utilisation d'une multiguanidine n'appartenant pas à la famille des hydroxydes comme agent actif de défrisage des fibres kératiniques.

21. Agent actif de défrisage des fibres kératiniques, au moyen d'une réaction de béta-élimination produisant de la déhydro-alanine et conduisant à la formation de lanthionine, comprenant au moins une multiguanidine n'appartenant pas à la famille des hydroxydes.

22. Utilisation en cosmétique des composés de formule (II).
